(19) Europäisches Patentamt
European Patent Office
Office européen des brevets

(11) Veröffentlichungsnummer: **0 177 710**
**A1**

(12) **EUROPÄISCHE PATENTANMELDUNG**

(21) Anmeldenummer: **85110066.9**

(22) Anmeldetag: **10.08.85**

(51) Int. Cl.⁴: **C 07 D 231/22**, C 07 D 231/18, C 07 D 231/20, C 07 D 239/34, C 07 D 285/08, C 07 D 513/04, C 07 D 249/12, C 07 D 271/06, C 07 D 233/70, C 07 D 263/38, C 07 D 277/34

(30) Priorität: **25.08.84 DE 3431272**

(43) Veröffentlichungstag der Anmeldung: **16.04.86 Patentblatt 86/16**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Kristen, Rolf, Dr., Carl-Langhansstrasse 27, D-4019 Monheim (DE)**
Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Köln 80 (DE)**
Erfinder: **Schmidt, Robert R. Dr., Im Waldwinkel 110, D-5060 Bergisch-Gladbach 2 (DE)**
Erfinder: **Santel, Hans Joachim, Dr., Gerstenkamp 19, D-5000 Köln (DE)**

(54) **Substituierte Phenylharnstoffe.**

(57) Neue substituierte Phenylharnstoffe der Formel

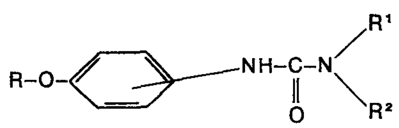

in welcher
R für gegebenenfalls substituierte Reste aus der Reihe Pyrimidinyl, Pyrazolyl, 1,2,4-Triazolyl, Thiazolo-[1,2,4]-triazolyl, 1,2,4-Oxadiazolyl, 1,2,4-Thiadiazolyl, Imidazolyl, 1,3-Oxazolyl oder 1,3-Thiazolyl steht,
$R^1$ für Wasserstoff oder Alkyl steht und
$R^2$ für Alkyl steht,
mehrere Verfahren zur Herstellung der neuen Stoffe und deren Verwendung als Herbizide.

EP 0 177 710 A1

BAYER AKTIENGESELLSCHAFT 5090 Leverkusen, Bayerwerk
Konzernverwaltung RP
Patentabteilung Dü/Ja-c
Ib

## Substituierte Phenylharnstoffe

Die vorliegende Erfindung betrifft neue substituierte Phenylharnstoffe, mehrere Verfahren zu ihrer Herstellung und ihre Verwendung als Herbizide.

Es ist bereits bekannt, daß bestimmte Phenylharnstoff-Derivate herbizide Eigenschaften besitzen (vgl. DE-AS 11 42 251). So kann zum Beispiel der 1,1-Dimethyl-3-/4-(4-chlorphenoxy)-phenyl/-harnstoff zur Bekämpfung von Unkraut eingesetzt werden. Die Wirkung dieses Stoffes ist jedoch vor allem bei niedrigen Aufwandmengen nicht immer ausreichend.

Es wurden nun neue substiuierte Phenylharnstoffe der Formel

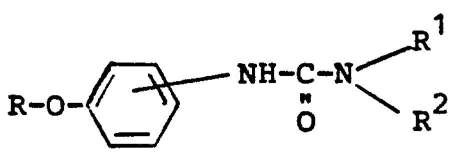

(I)

in welcher

R für gegebenenfalls substituierte Reste aus der Reihe Pyrimidinyl, Pyrazolyl, 1,2,4-Triazolyl,

Thiazolo-[1,2,4]-triazolyl, 1,2,4-Oxadiazolyl, 1,2,4-Thiadiazolyl, Imidazolyl, 1,3-Oxazolyl oder 1,3-Thiazolyl steht,

$R^1$ für Wasserstoff oder Alkyl steht und

$R^2$ für Alkyl steht,

gefunden.

Weiterhin wurde gefunden, daß man neue substituierte Phenylharnstoffe der Formel (I) erhält, wenn man

a) substituierte Phenylamine der Formel

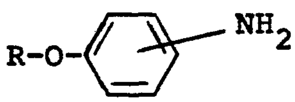

(II)

in welcher

R die oben angegebene Bedeutung hat,

entweder

α) mit Carbamoylhalogeniden der Formel

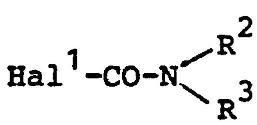

(III)

in welcher

$R^2$ die oben angegebene Bedeutung hat,

$R^3$ für Alkyl steht und

$Hal^1$ für Halogen steht,

gegebenenfalls in Gegenwart eines Säureakzeptors und gegebenenfalls in Gegenwart eines Verdünnungsmittels umsetzt,

oder

ß) mit Isocyanaten der Formel

$R^2$-NCO (IV)

in welcher

$R^2$ die oben angegebene Bedeutung hat,

gegebenenfalls in Gegenwart eines Katalysators und gegebenenfalls in Gegenwart eines Verdünnungsmittels umsetzt,

oder

b) Carbamidsäure-Derivate der Formel

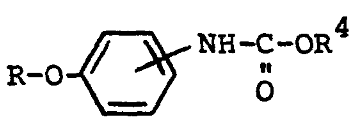

(V)

in welcher

R die oben angegebene Bedeutung hat und

$R^4$ für gegebenenfalls substituiertes Alkyl oder gegebenenfalls substituiertes Phenyl steht,

mit Aminen der Formel

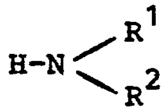

(VI)

in welcher

$R^1$ und $R^2$ die oben angegebene Bedeutung haben,

gegebenenfalls in Gegenwart eines Verdünnungsmittels umsetzt,

oder

c) substituierte Phenylamine der Formel

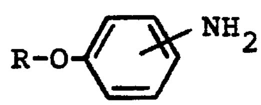

(II)

in welcher

R die oben angegebene Bedeutung hat,

zunächst mit Phosgen in Gegenwart eines Verdünnungsmittels umsetzt und die dabei entstehenden Isocyanate der Formel

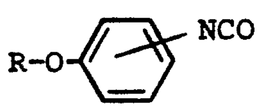

(VII)

in welcher

R die oben angegebene Bedeutung hat,

mit Aminen der Formel

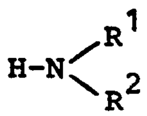

(VI)

in welcher

$R^1$ und $R^2$ die oben angegebene Bedeutung haben,

gegebenenfalls in Gegenwart eines Katalysators und gegebenenfalls in Gegenwart eines Verdünnungsmittels umsetzt.

Schließlich wurde gefunden, daß sich die neuen substituierten Phenylharnstoffe der Formel (I) durch eine hervorragende herbizide Wirksamkeit auszeichnen.

Überraschenderweise besitzen die erfindungsgemäßen substituierten Phenylharnstoffe der Formel (I) wesentlich bessere selektive herbizide Eigenschaften als der 1,1-Dimethyl-3-/4-(4-chlorphenoxy)-phenyl/-harnstoff, welches ein konstitutionell ähnlicher, vorbekannter Wirkstoff gleicher Wirkungsart ist.

Die erfindungsgemäßen substitierten Phenylharnstoffe sind durch die Formel (I) allgemein definiert. In dieser Formel steht R vorzugsweise für gegebenenfalls einfach oder mehrfach, gleichartig oder verschieden substituiertes 5-Pyrimidinyl oder 6-Pyrimidinyl, wobei

gegebenenfalls substituiertes Alkyl mit 1 bis 6 Kohlenstoffatomen, Alkoxy mit 1 bis 6 Kohlenstoffatomen, Alkylthio mit 1 bis 6 Kohlenstoffatomen, Cycloalkyl mit 3 bis 8 Kohlenstoffatomen, Amino, Alkylamino mit 1 bis 4 Kohlenstoffatomen, Dialkylamino mit 1 bis 4 Kohlenstoffatomen in jeder Alkylgruppe und/oder gegebenenfalls substituiertes Phenyl als Substituenten in Frage kommen. R steht weiterhin vorzugsweise für Pyrazolyl, das einfach oder zweifach, gleichartig oder verschieden substituiert sein kann durch Alkyl mit 1 bis 4 Kohlenstoffatomen, Cycloalkyl mit 3 bis 8 Kohlenstoffatomen und/oder Phenyl, welches seinerseits einfach bis dreifach, gleichartig oder verschieden substituiert sein kann durch Alkyl mit 1 oder 2 Kohlenstoffatomen, Alkoxy mit 1 oder 2 Kohlenstoffatomen, Fluor, Chlor, Brom, Trihalogenmethyl, Trihalogenmethoxy, Dihalogenmethyl und/oder Trihalogenmethylsulfonyl. R steht ferner vorzugsweise für 1,2,4-Triazolyl oder Thiazolo-[1,2,4]-triazolyl, wobei jeder dieser Reste einfach oder zweifach, gleichartig oder verschieden substituiert sein kann durch Alkyl mit 1 bis 4 Kohlenstoffatomen und/oder gegebenenfalls substituiertes Phenyl. Außerdem steht R vorzugsweise für 1,2,4-Oxadiazolyl, 1,2,4-Thiadiazolyl, Imidazolyl, 1,3-Oxazolyl oder 1,3-Thiazolyl, wobei jeder dieser Reste einfach bis dreifach, gleichartig oder verschieden substituiert sein kann durch Alkyl mit 1 bis 4 Kohlenstoffatomen und/oder Halogen, wie zum Beispiel Chlor oder Brom. $R^1$ steht vorzugsweise für Wasserstoff oder Alkyl mit 1 bis 6 Kohlenstoffatomen, und $R^2$ steht vorzugsweise für Alkyl mit 1 bis 6 Kohlenstoffatomen.

Besonders bevorzugt sind diejenigen substituierten Phenylharnstoffe der Formel (I), in denen

R für gegebenenfalls einfach bis dreifach durch Methyl, Ethyl, n-Propyl, iso-Propyl, n-Butyl, iso-Butyl, sek.-Butyl, tert.-Butyl, Methoxy, Ethoxy, Methylthio, Ethylthio, Cyclopropyl, Cyclopentyl, Cyclohexyl, Amino, Methylamino, Dimethylamino, Ethylamino, Diethylamino, n-Propylamino, Di-n-propylamino, iso-Propylamino, Di-iso-propylamino, n-Butylamino, Di-n-butylamino, iso-Butylamino, Di-iso-butylamino und/oder Phenyl substituiertes 5-Pyrimidinyl oder 6-Pyrimidinyl steht, oder

R für Pyrazolyl steht, welches einfach oder zweifach, gleichartig oder verschieden substituiert sein kann durch Methyl, Ethyl, n-Propyl, iso-Propyl, n-Butyl, iso-Butyl, sek.-Butyl, tert.-Butyl, Trifluormethyl, Trichlormethyl, Difluormethyl, Dichlormethyl und/oder Phenyl, welches seinerseits einfach bis dreifach, gleichartig oder verschieden durch Methyl, Methoxy, Trifluormethyl, Trifluormethoxy, Difluormethyl, Trifluormethylsulfonyl, Chlor und/oder Brom substituiert sein kann, oder

R für 1,2,4-Triazolyl oder Thiazolo-[1,2,4]-triazolyl steht, wobei jeder dieser Reste einfach oder zweifach, gleichartig oder verschieden durch Methyl, Ethyl, n-Propyl, iso-Propyl und/oder Phenyl substituiert sein kann, oder

R für 1,2,4-Oxadiazolyl, 1,2,4-Thiadiazolyl, Imidazolyl, 1,3-Oxazolyl oder 1,3-Thiazolyl steht, wobei jeder dieser Reste einfach oder zweifach, gleichartig oder verschieden substituiert sein kann durch Methyl, Ethyl, n-Propyl, iso-Propyl, Chlor und/oder Brom,

$R^1$ für Wasserstoff, Methyl oder Ethyl steht und

$R^2$ für Methyl oder Ethyl steht.

Verwendet man 3-(2-Methyl-pyrazol-4-yl-oxy)-anilin und N,N-Dimethylcarbamoylchlorid als Ausgangsstoffe, so kann der Verlauf des erfindungsgemäßen Verfahrens (a, Variante α) durch das folgende Formelschema wiedergegeben werden:

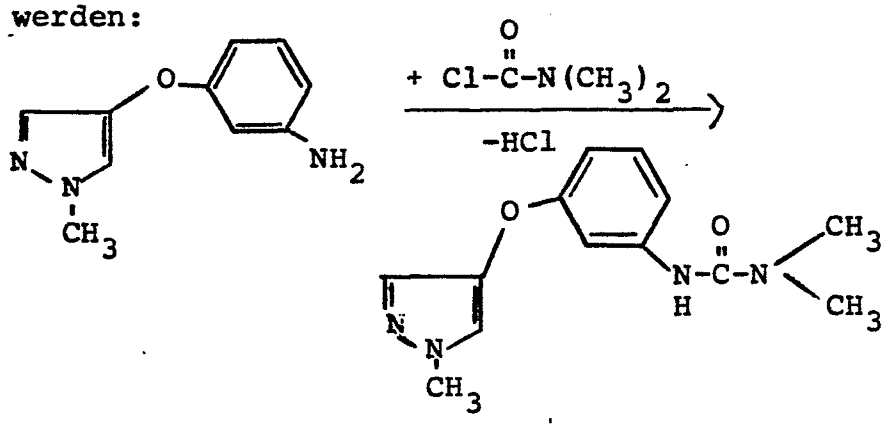


Verwendet man 3-(2-Ethyl-pyrimidin-5-yloxy)-anilin und Methylisocyanat als Ausgangsstoffe, so kann der Verlauf des erfindungsgemäßen Verfahrens (a, Variante ß) durch das folgende Formelschema wiedergegeben werden:

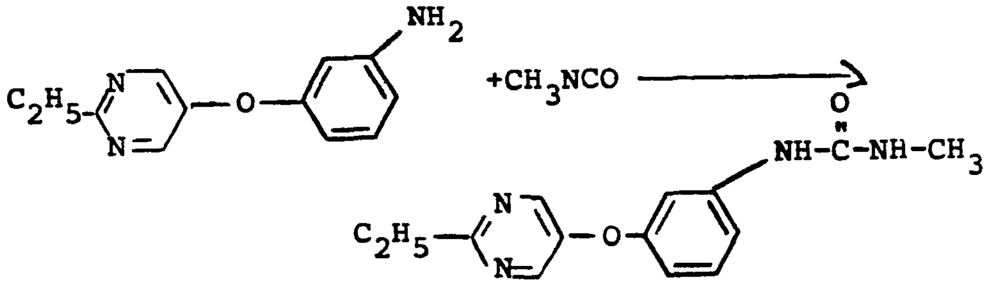


Verwendet man N-/4-(1-Ethyl-3-iso-propyl-pyrazol-5-yloxy)-phenyl/-carbamidsäure-phenylester und Diethylamin als Ausgangsstoffe, so kann der Verlauf des erfindungsgemäßen Verfahrens (b) durch das folgende Formelschema wiedergegeben werden:

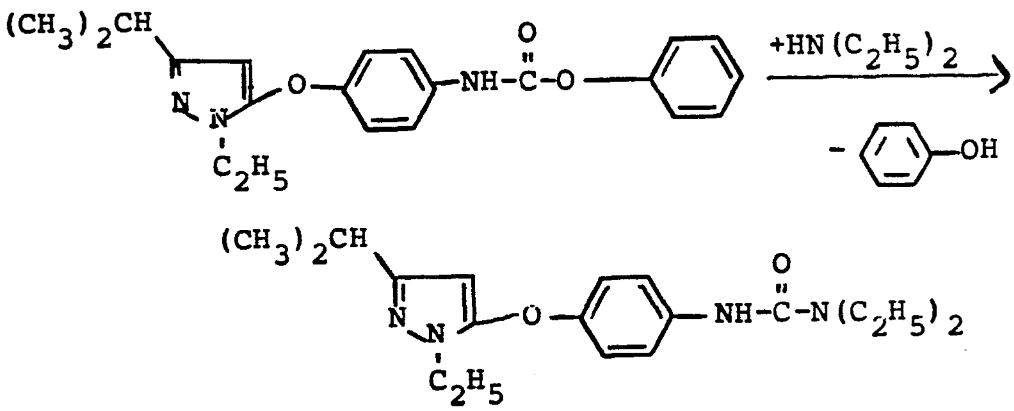


Verwendet man 3-(2-Ethyl-pyrazol-4-yl-oxy)-anilin und Phosgen als Ausgangsstoffe und Diethylamin als Reaktionskomponente, so kann der Verlauf des erfindungsgemäßen Verfahrens (c) durch das folgende Formelschema wiedergegeben werden:

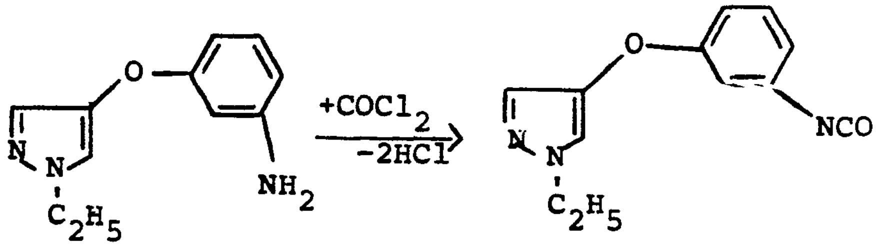

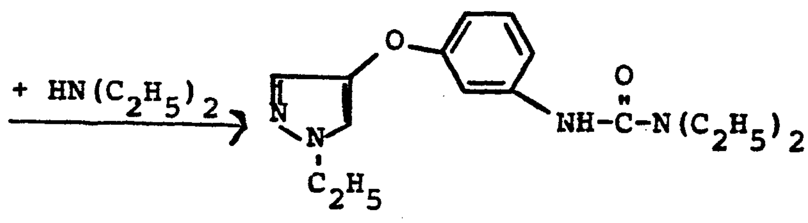

Die bei dem erfindungsgemäßen Verfahren (a, Varianten α und β) als Ausgangsstoffe benötigten substituierten Phenylamine sind durch die Formel (II) definiert. In dieser Formel hat R vorzugsweise diejenigen Bedeutungen, die im Zusammenhang mit der Beschreibung der erfindungsgemäßen Stoffe der Formel (I) vorzugsweise für diesen Rest genannt wurden.

Als Beispiele für substituierte Phenylamine der Formel (II) seien die in der folgenden Tabelle aufgeführten Stoffe genannt:

Tabelle 1

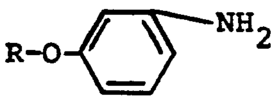

(IIa)

;

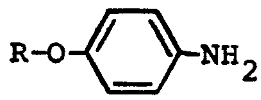

(IIb)

;

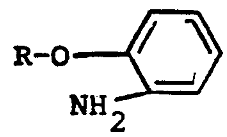

(IIc)

| R | R | R |
|---|---|---|
| 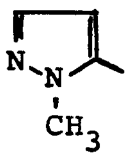 | 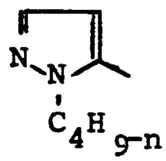 | 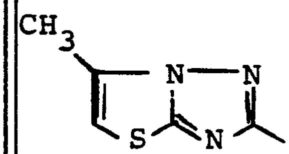 |
| 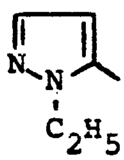 | 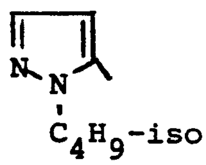 | 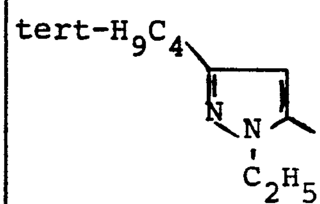 |
| 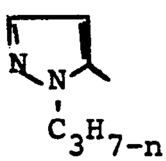 | 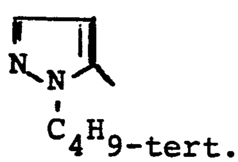 | 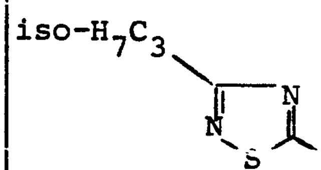 |
| 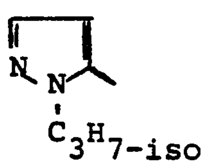 | 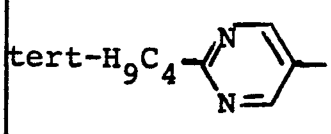 | 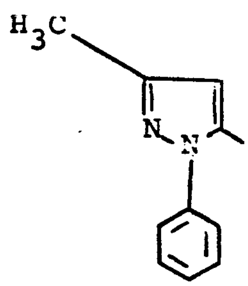 |

Tabelle 1 (Fortsetzung)

| R | R |
|---|---|
| 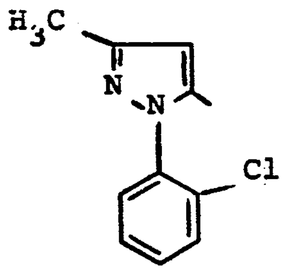 | 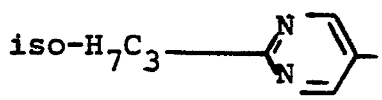 |
| 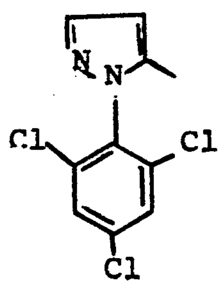 | 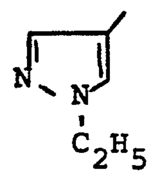 |
| 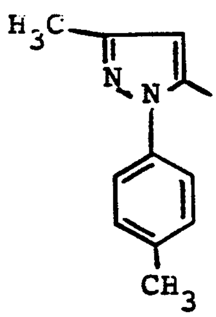 | 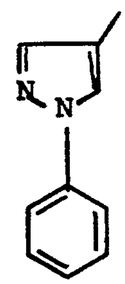 |
| 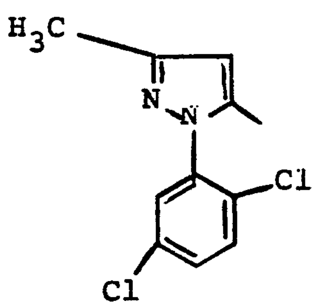 | 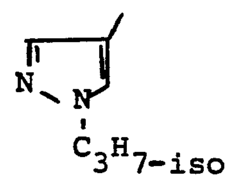 |
| 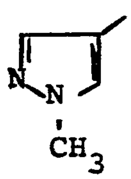 | 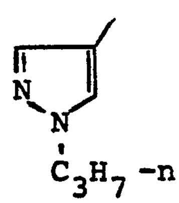 |

Die substituierten Phenylamine der Formel (II) sind bisher nur teilweise bekannt. Sie lassen sich jedoch in einfacher Weise nach im Prinzip bekannten Verfahren herstellen. So erhält man Verbindungen der Formel (II), indem man

d) Halogenverbindungen der Formel

$$R\text{-}Hal^2 \qquad (VIII)$$

in welcher

R die oben angegebene Bedeutung hat und
$Hal^2$ für Chlor oder Brom steht,

mit Aminophenolen der Formel

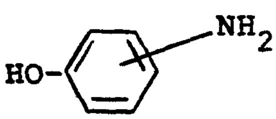

(IX)

gegebenenfalls in Gegenwart eines Säureakzeptors, wie zum Beispiel Natrium- oder Kaliumhydroxid, und gegebenenfalls in Gegenwart eines Verdünnungsmittels, wie zum Beispiel Dimethylsulfoxid, bei Temperaturen zwischen 50 °C und 160°C umsetzt

oder

e) Hydroxylverbindungen der Formel

$$R\text{-}OH \qquad (X)$$

in welcher

R die oben angegebene Bedeutung hat,

mit Chlornitrobenzolen der Formel

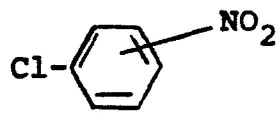

(XI)

gegebenenfalls in Gegenwart eines Säureakzeptors, wie zum Beispiel Natrium- oder Kalium-hydroxid, und gegebenenfalls in Gegenwart eines Verdünnungsmittels, wie zum Beispiel Dimethylsulfoxid, bei Temperaturen zwischen 50°C und 160°C umsetzt und die auf diese Weise entstandenen Nitrophenyl-Derivate der Formel

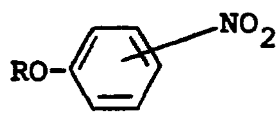

(XII)

in welcher,

R die oben angegebene Bedeutung hat,

mit Wasserstoff in Gegenwart eines Katalysators, wie zum Beispiel Raney-Nickel, und in Gegenwart eines Verdünnungsmittels, wie zum Beispiel Methanol oder Ethanol, bei Temperaturen zwischen 0°C und 120 °C und gegebenenfalls unter erhöhtem Druck, reduziert.

Die für die Herstellung von substituierten Phenylaminen der Formel (II) nach den obigen Verfahren (d) und (e) als Ausgangsstoffe benötigten Verbindungen der Formeln (VIII), (IX), (X) und (XI) sowie die Zwischenprodukte der Formel (XII) sind bekannt oder lassen sich nach

bekannten Methoden herstellen (vgl. DE-OS 1 942 015, DE-OS 2 643 262, DE-OS 2 706 127, DE-OS 3 023 675 und DE-OS 2 912 494).

Bei der Durchführung der Verfahren (d) und (e) erfolgt die Aufarbeitung und die Isolierung der Verbindungen der Formel (II) nach üblichen Methoden.

Die bei dem erfindungsgemäßen Verfahren (a, Variante $\alpha$) als Reaktionskomponenten benötigten Carbamoylhalogenide sind durch die Formel (III) definiert. In dieser Formel steht $R^2$ vorzugsweise für diejenigen Reste, die bereits im Zusammenhang mit der Beschreibung der erfindungsgemäßen Stoffe der Formel (I) vorzugsweise für diesen Substituenten genannt wurden. $R^3$ steht vorzugsweise für Alkyl mit 1 bis 6 Kohlenstoffatomen, und $Hal^1$ steht vorzugsweise für Chlor oder Brom.

Als Beispiele für Carbamoylhalogenide seien genannt: Dimethyl-, Diethyl-, Di-n-propyl-, Di-iso-propyl-, Di-n-butyl-, Di-iso-butyl-, Di-sek-butyl, Ethyl-methyl-, n-Propyl-methyl-, iso-Propyl-methyl-, n-Butyl-methyl, iso-Butyl-methyl-, sek-Butyl-methyl-, tert.Butyl-methyl-, n-Propyl-ethyl-, iso-Propyl-ethyl-, n-Butyl-ethyl-, iso-Butyl-ethyl-, sek.Butyl-ethyl-, tert.Butyl-ethyl-, iso-Propyl-n-propyl-, n-Butyl-n-propyl-, iso-Butyl-n-propyl-, sek.Butyl-n-propyl-, tert.-Butyl-n-propyl-, n-Butyl-iso-propyl-, iso-Butyl-iso-propyl-, iso-Butyl-n-butyl-, sek.-Butyl-n-butyl- und tert.Butyl-n-butyl-carbamoylchlorid bzw. -bromid.

Die Carbamoylhalogenide der Formel (III) sind allgemein bekannte Verbindungen der organischen Chemie.

Die bei dem erfindungsgemäßen Verfahren (a, Variante ß) als Reaktionskomponenten benötigten Isocyanate sind durch die Formel (IV) definiert. In dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Rest genannt wurden.

Als Beispiele für Isocyanate der Formel (IV) seien genannt: Methyl-, Ethyl-, n-Propyl-, iso-Propyl-, n-Butyl-, iso-Butyl-, sek-Butyl- und tert.-Butyl-isocyanat.

Die Isocyanate der Formel (IV) sind allgemein bekannte Verbindungen der organischen Chemie.

Die Umsetzungen nach dem erfindungsgemäßen Verfahren (a) werden sowohl bei der Variante α als auch bei der Variante ß vorzugsweise in Gegenwart eines Verdünnungsmittels durchgeführt. Als Verdünnungsmittel kommen hierbei praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone,

wie Aceton, Methyl-ethyl-, Methyl-isopropyl- und Methylisobutylketon, Ester, wie Essigsäure-methylester und -ethylester, Nitrile, wie z. B. Acetonitril und Propionitril, Amide, wie z. B. Dimethylformamid, Dimethylacetamid und N-Methyl-pyrrolidon, sowie Dimethylsulfoxid, Tetramethylensulfon, Hexamethylphosphorsäuretriamid und Pyridin.

Als Säureakzeptoren können bei dem erfindungsgemäßen Verfahren (a, Variante α) alle üblicherweise für derartige Umsetzungen verwendbaren Säurebindemittel eingesetzt werden. Vorzugsweise in Frage kommen Alkalicarbonate wie Natrium- und Kaliumcarbonat, ferner aliphatische, aromatische oder heterocyclische Amine, beispielsweise Triethylamin, Trimethylamin, Dimethylanilin, Dimethylbenzylamin, 1,5-Diazabicyclo[4,3,0]non-5-en (DBN), 1,8-Diazabicyclo[5,4,0]undec-7-en (DBN), 1,4-Diazatricyclo[2,2,2]octan, Pyridin, Chinolin oder Piperidin. Verwendet man flüssige Amine als Säureakzeptoren, so können diese gleichzeitig als Lösungsmittel fungieren, sofern man sie in einem ausreichenden Überschuß einsetzt.

Als Katalysatoren kommen bei der Durchführung des erfindungsgemäßen Verfahrens (a, Variante ß) alle für derartige Umsetzungen üblichen Reaktionsbeschleuniger in Betracht. Vorzugsweise verwendbar sind basische Katalysatoren, wie Triethylamin, Trimethylamin, Dimethylanilin, Dimethylbenzylamin, 1,5-Diazabicyclo[4,3,0]non-5-en (DBN), 1,8-Diazabicyclo[5,4,0]undec-7-en (DBN), 1,4-Diazatricyclo[2,2,2]octan und Pyridin.

Die Reaktionstemperaturen können bei der Umsetzung nach dem erfindungsgemäßen Verfahren (a) sowohl in der Variante α als auch in der Variante β innerhalb eines größeren Bereiches variiert werden. Im allgemeinen arbeitet man in der Variante α bei Temperaturen zwischen -20°C und +160°C, vorzugsweise zwischen 0°C und +120°C, und in der Variante β bei Temperaturen zwischen 0°C und +150°C, vorzugsweise zwischen 40°C und 120°C. Die Umsetzung nach dem erfindungsgemäßen Verfahren (a) wird sowohl in der Variante α als auch in der Variante β im allgemeinen unter Normaldruck durchgeführt. Es ist jedoch auch möglich, insbesondere bei der Verwendung von gasförmigen Ausgangsstoffen, unter erhöhtem Druck zu arbeiten.

Bei der Durchführung des erfindungsgemäßen Verfahrens (a, Varianten α und β) werden die Ausgangsstoffe der Formeln (II) und (III) bzw. (II) und (IV) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in Gegenwart eines geeigneten Verdünnungsmittels und in Gegenwart eines Säurebindemittels bzw. eines Katalysators durchgeführt. Das Reaktionsgemisch wird dabei im allgemeinen mehrere Stunden bei der jeweils erforderlichen Temperatur gerührt und dann nach üblichen Methoden aufgearbeitet.

Die bei dem erfindungsgemäßen Verfahren (b) als Ausgangsstoffe benötigten Carbamidsäure-Derivate sind durch die Formel (V) definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden. $R^4$ steht vorzugsweise für Alkyl mit 1 bis 6 Kohlenstoffatomen oder Phenyl. Insbesondere steht $R^4$ für Methyl, Ethyl, n-Propyl, iso-Propyl, n-Butyl oder Phenyl.

Als Beispiele für Carbamidsäure-Derivate der Formel (V) seien die in der folgenden Tabelle 2 aufgeführten Verbindungen genannt:

<u>Tabelle 2</u>

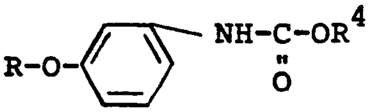

(Va)

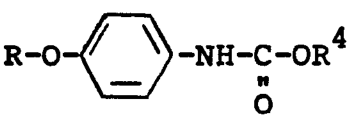

(Vb)

und

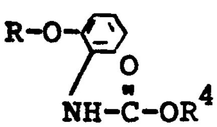

(Vc)

| R | $R^4$ |
|---|---|
| 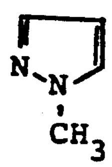 | $CH_3$ |
| " | $C_2H_5$ |
| " | 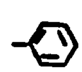 |
| 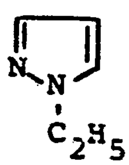 | $CH_3$ |

Tabelle 2 (Fortsetzung)

| R | $R^4$ |
|---|---|
| 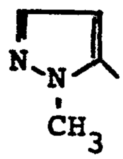 | $C_2H_5$ |
| " | 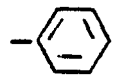 |
| 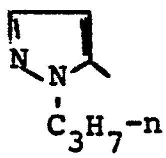 | $CH_3$ |
| " | $C_2H_5$ |
| " | 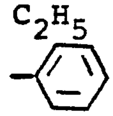 |
| 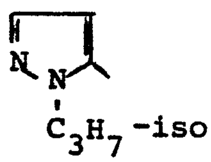 | $CH_3$ |
| " | $C_2H_5$ |
| " | 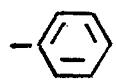 |
| 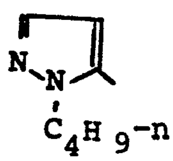 | $CH_3$ |
| " | $C_2H_5$ |
| " | 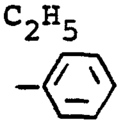 |
| 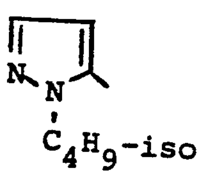 | $CH_3$ |
| " | $C_2H_5$ |
| " | 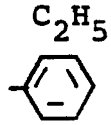 |

Tabelle 2 (Fortsetzung)

| R | $R^4$ |
|---|---|
| 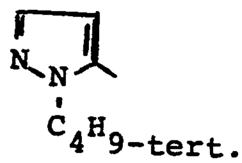 | $CH_3$ |
| " | $C_2H_5$ |
| " | 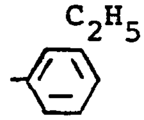 |
| 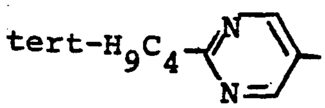 | $CH_3$ |
| " | $C_2H_5$ |
| " | 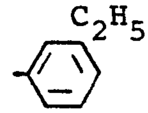 |
| 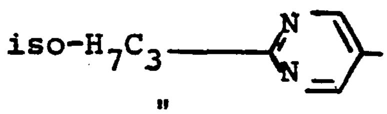 | $CH_3$ |
| " | $C_2H_5$ |
| " | 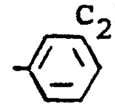 |
| 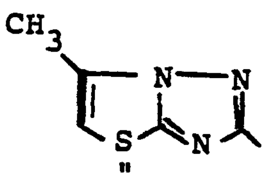 | $CH_3$ |
| " | $C_2H_5$ |
| " | 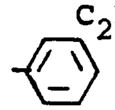 |
| 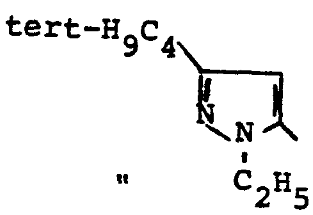 | $CH_3$ |
| " | $C_2H_5$ |
| " | 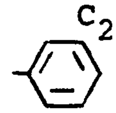 |
| 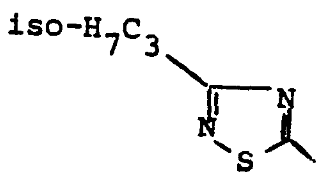 | $CH_3$ |

Tabelle 2 (Fortsetzung)

| R | $R^4$ |
|---|---|
| 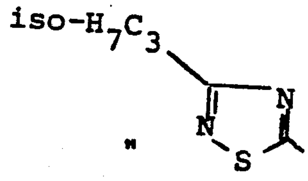 | $C_2H_5$ |
| " | 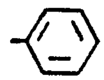 |
| 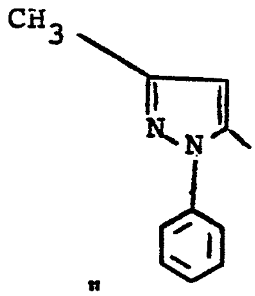 | $CH_3$ |
| " | $C_2H_5$ |
| " | 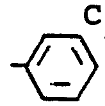 |
| 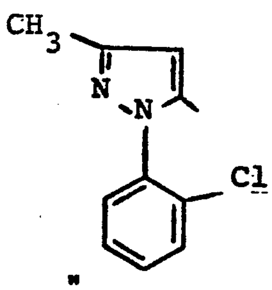 | $CH_3$ |
| " | $C_2H_5$ |
| " | 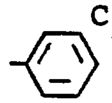 |
| 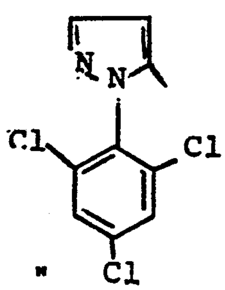 | $CH_3$ |
| " | $C_2H_5$ |
| " | 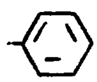 |

Tabelle 2 (Fortsetzung)

| R | $R^4$ |
|---|---|
| 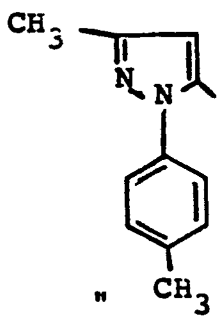 | $CH_3$ |
| " | $C_2H_5$ |
| " | 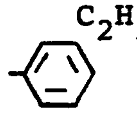 |
| 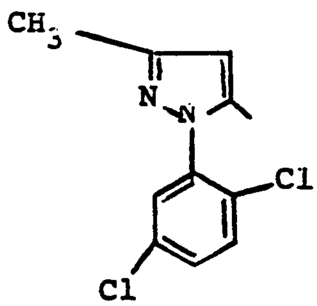 | $CH_3$ |
| " | $C_2H_5$ |
| " | 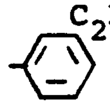 |
| 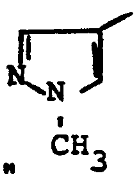 | $CH_3$ |
| " | $C_2H_5$ |
| " | 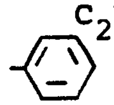 |
| 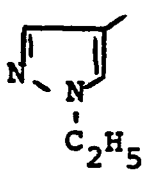 | $CH_3$ |
| " | $C_2H_5$ |
| " | 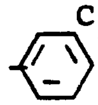 |

Tabelle 2 (Fortsetzung)

| R | $R^4$ |
|---|---|
| 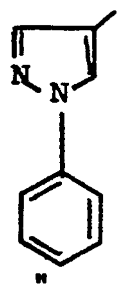 | $CH_3$ |
| " | |
| " | 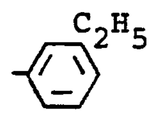 |
| 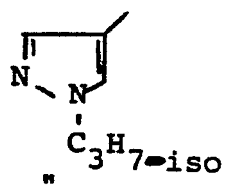 | $CH_3$ |
| " | |
| " | 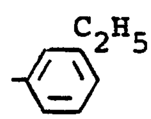 |
| 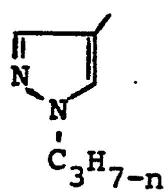 | $CH_3$ |
| " | |
| " | 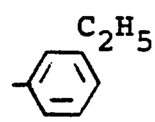 |

Die Carbamidsäure-Derivate der Formel (V) sind zum Teil noch nicht bekannt. Sie lassen sich jedoch in einfacher Weise aus substituierten Phenylaminen der Formel (II) nach bekannten Verfahren herstellen (vgl. Houben-Weyl-Müller "Methoden der organischen Chemie" Band VIII, Seite 137 ff. und Band E4, Seite 144 ff, Thieme-Verlag, Stuttgart, sowie Herstellungsbeispiele).

Die bei dem erfindungsgemäßen Verfahren (b) als Reaktionskomponenten benötigten Amine sind durch die Formel (VI) definiert. In dieser Formel haben $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Amine der Formel (VI) seien genannt: (Di)Methyl-, (Di)Ethyl-, (Di)n-Propyl-, (Di)iso-Propyl-, (Di)n-Butyl-, (Di)-iso-Butyl-, (Di)-sek.-Butyl-, (Di)-tert.Butyl-, Ethyl-methyl-, n-Propyl-methyl-, iso-Propyl-methyl-, n-Butyl-methyl-, iso-Butyl-methyl-, sek.-Butyl-methyl-, tert.-Butyl-methyl-, n-Propyl-ethyl-, iso-Propyl-ethyl-, n-Butyl-ethyl-, iso-Butyl-ethyl-, sek-Butyl-ethyl-, tert.Butyl-ethyl-, iso-Propyl-n-propyl-, n-Butyl-n-propyl-, iso-Butyl-n-propyl-, sek-Butyl-n-propyl-, tert.-Butyl-n-propyl-, n-Butyl-iso-propyl-, iso-Butyl-iso-propyl, iso-Butyl-n-butyl-, sek.-Butyl-n-butyl- und tert.- Butyl-n-butyl-amin.

Die Verbindungen der Formel (VI) sind allgemein bekannte Verbindungen der organischen Chemie.

Als Verdünnungsmittel kommen bei der Durchführung des erfindungsgemäßen Verfahrens (b) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton.

Außerdem können auch Alkohole, wie Methanol oder Ethanol eingesetzt werden.

Das erfindungsgemäße Verfahren (b) wird im allgemeinen bei Temperaturen zwischen 0°C und 80°C durchgeführt. Bevorzugt wird der Bereich zwischen 0°C und 50°C. Die Umsetzungen können sowohl unter Normaldruck als auch unter erhöhtem Druck durchgeführt werden.

Zur Durchführung des erfindungsgemäßen Verfahrens (b) werden die Amine der Formel (VI) im allgemeinen im Überschuß eingesetzt. Bei der Verwendung von gasförmigen Aminen der Formel (VI) können diese Amine durch das Gemisch aus Verdünnungsmittel und Verbindungen der Formel (V) geleitet werden. Die Aufarbeitung der Reaktionsprodukte geschieht nach üblichen Methoden.

Als Katalysatoren kommen bei der Durchführung des erfindungsgemäßen Verfahrens (c) alle für derartige Umsetzungen üblichen basischen Reaktionsbeschleuniger in Betracht. Vorzugsweise in Frage kommen diejenigen Katalysatoren, die bereits im Zusammenhang mit der Beschreibung des erfindungsgemäßen Verfahrens (a, Variante ß) vorzugsweise genannt wurden.

Als Verdünnungsmittel kommen bei der Durchführung des erfindungsgemäßen Verfahrens (c) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton.

Die Reaktionstemperaturen können bei dem erfindungsgemäßen Verfahren (c) innerhalb eines größeren Bereiches variiert werden. In der ersten Stufe arbeitet man im allgemeinen bei Temperaturen zwischen 30°C und 160°C, vorzugsweise zwischen 60°C und 130°C. In der zweiten Stufe arbeitet man im allgemeinen bei Temperaturen zwischen 0°C und 150°C, vorzugsweise zwischen 15°C und 110°C. Die Umsetzungen werden im allgemeinen unter Normaldruck durchgeführt.

Bei der Durchführung des erfindungsgemäßen Verfahrens (c) setzt man substituierte Phenylamine der Formel (II) in der ersten Stufe mit einem Überschuß an Phosgen um. Nach Beendigung der Umsetzung wird überschüssiges Phosgen entfernt, und die entstandenen Isocyanate der Formel (VII) werden entweder direkt oder nach vorheriger Isolierung mit einem Überschuß an Aminen der Formel (VI) umgesetzt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden.

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-,Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können vorzugsweise zur selektiven Bekämpfung mono- und dikotyler Unkräuter, vorzugsweise in monokotylen Kulturen, wie z. B. Mais, Getreide und Reis, eingesetzt werden. Die erfindungsgemäßen Wirkstoffe können auch zur Bekämpfung von Reiskrankheiten, wie Pyricularia oryzae, eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie

Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch als Mischungen mit bekannten Herbiziden zur Unkrautbekämpfung Verwendung finden, wobei Fertigformulierung oder Tankmischung möglich ist.

Für die Mischungen kommen bekannte Herbizide wie z. B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie zum Beispiel Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher

Weise, z. B. durch Gießen, Spritzen, Sprühen, Streuen.

Die erfindungsgemäßen Wirkstoffe können sowohl vor als auch insbesondere nach dem Auflaufen der Pflanzen appliziert werden.

Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,01 und 10 kg Wirkstoff pro ha, vorzugsweise zwischen 0,05 und 5 kg/ha.

Die Herstellung und die Verwendung der erfindungsgemäßen Stoffe geht aus den nachfolgenden Beispielen hervor.

Herstellungsbeispiele

Beispiel 1

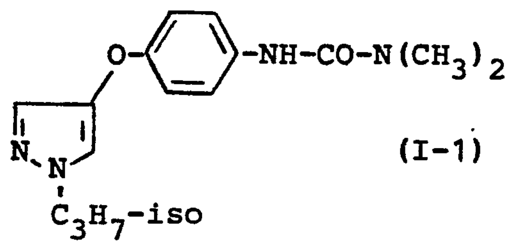

(I-1)

(Verfahren a, Variante α)

Zu 10,9 g (0,05 Mol) 4-(2-Isopropyl-pyrazol-4-yloxy)-anilin in 20 ml Pyridin wurden bei 20°C 6 g (0,05 Mol) Dimethylcarbamoylchlorid unter Rühren in der Weise zugetropft, daß eine Reaktionstemperatur von 30°C nicht überschritten wurde. Dann wurde das Reaktionsgemisch zweimal mit jeweils 100 ml Wasser digeriert. Das zurückbleibende Öl wurde mit 3 ml Toluol verrieben. Die sich bildenden Kristalle wurden abgesaugt und getrocknet.

Man erhielt 10,7 g (74 % der Theorie) 3,3-Dimethyl-1-[4-(2-i-propyl-pyrazol-4-yloxy)-phenyl]-harnstoff in Form farbloser Kristalle vom Schmelzpunkt 116°C.

Beispiel 2

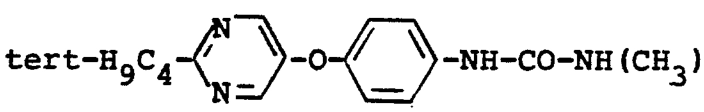

(Verfahren a, Variante ß) (I-2)

6 g (0,0247 Mol) 4-(2-tert-Butylpyrimidin-5-yloxy)-anilin wurden in 80 ml Toluol mit 1,6 g (0,028 Mol) Methylisocyanat und 3 Tropfen Triethylamin bei 65°C-70°C gerührt. Nach 3 Stunden wurde noch 1 g Methylisocyanat zugegeben und eine weitere Stunde bei 65°C-70°C gerührt. Dann wurde der Ansatz auf 0°C-3°C abgekühlt, die ausgefallene Substanz abgesaugt, mit Toluol und anschließend mit Ether gewaschen und bei 60°C getrocknet.

Man erhielt 6,6 g (89 % der Theorie) 1-/4-(2-tert- Butylpyrimidin-5-yloxy)-phenyl7-3-methyl-harnstoff vom Schmelzpunkt 164 - 166°C.

Beispiel 3

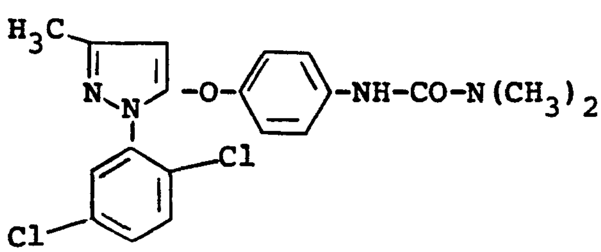

(I-3)

(Verfahren b)

6 g (0,0132 Mol) N-4-/1-(2,5-Dichlorphenyl)-3-methyl-pyrazol-5-yloxy7-phenylcarbamidsäurephenylester wurden in 20 ml 33 %iger ethanolischer Dimethylaminlösung 3 Stunden bei 20°C gerührt. Danach wurden das Lösungsmittel sowie der Überschuß an Amin unter vermindertem Druck entfernt. Das zurückbleibende Öl wurde in Methylenchlorid gelöst, mit 0,5 N wäßriger Natronlauge und Wasser gewaschen und über Natriumsulfat getrocknet. Die organische Phase wurde anschließend eingeengt.

Man erhielt 4,8 g (90 % der Theorie) 3,3-Dimethyl-1-{4-/1-(2,5-dichlorphenyl)-3-methyl-pyrazol-5-yloxy/-phenyl}-harnstoff in Form farbloser Kristalle vom Schmelzpunkt 64°C - 70°C.

Nach den in den Beispielen 1 bis 3 angegebenen Methoden wurden auch die in den folgenden Beispielen aufgeführten erfindungsgemäßen Stoffe hergestellt.

Beispiel 4

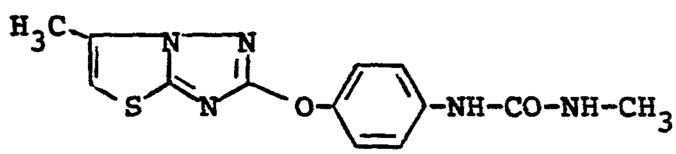

(I-4)

Fp: 205°C - 207°C

Beispiel 5

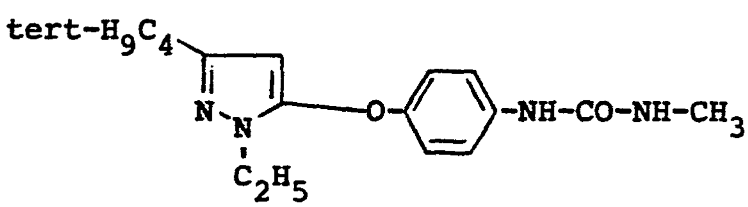

(I-5)

Fp: 206°C - 207°C

Beispiel 6

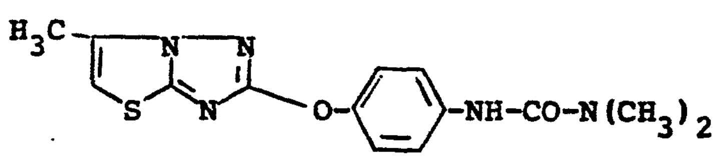

(I-6)

Fp: 142°C - 145°C

Beispiel 7

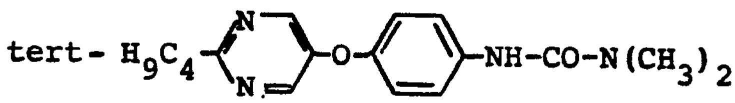

(I-7)

Fp: 176°C - 177°C

Beispiel 8

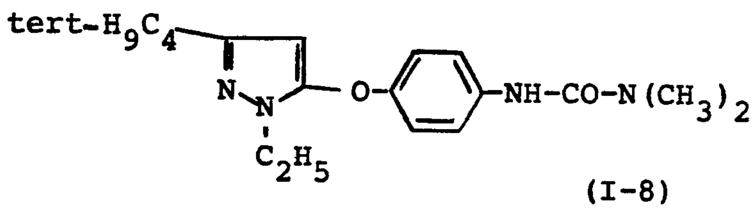

(I-8)

Fp: 172°C

Beispiel 9

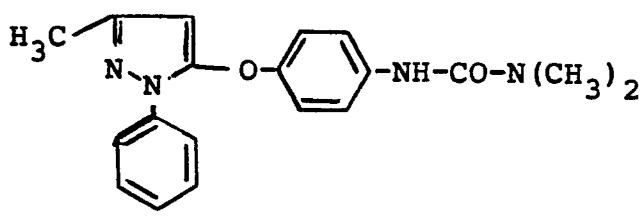

(I-9)

Fp: 123°C - 127°C

Beispiel 10

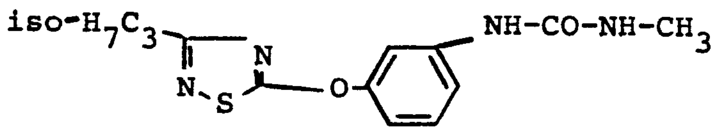

(I-10)

Fp: 99°C - 100°C

Beispiel 11

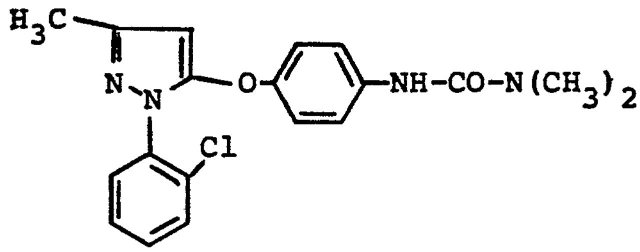

(I-11)

Fp: 146°C - 148°C

Beispiel 12

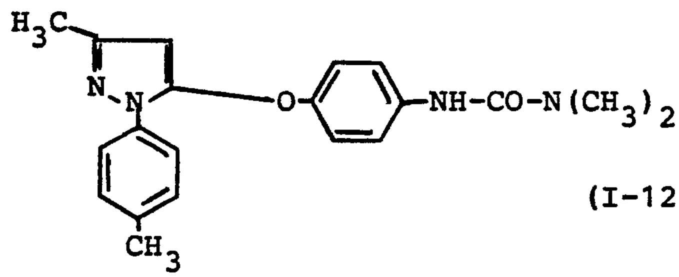

(I-12)

Fp: 191°C - 193°C

Beispiel 13

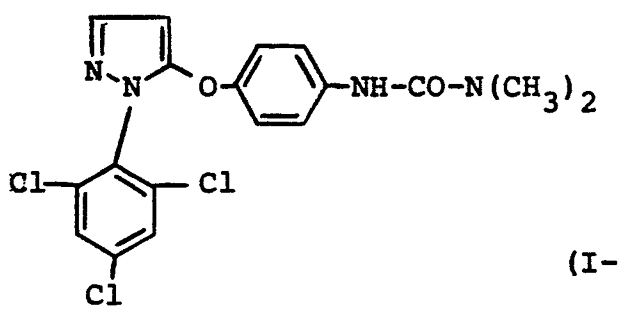

(I-13)

Vorprodukte der Formel (II)

Beispiel 14

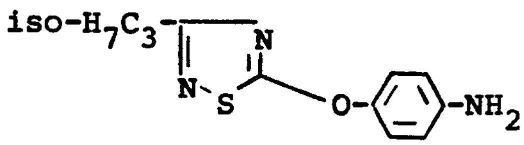

(II-1)

32,9 g (0,3 Mol) 4-Aminophenol wurden in 150 ml Dimethylsulfoxid mit 12 g Natriumhydroxidplätzchen 1 Stunde bei 80°C gerührt. Anschließend wurden 48 g (0,295 Mol) 5-Chlor-3-iso-propyl-1,2,4-thiadiazol bei 80°C - 100°C zugetropft, und es wurde weitere 6 Stunden bei 80°C - 90°C nachgerührt. Danach wurden 80 ml Wasser und 80 ml Methylenchlorid zugefügt. Anschließend wurde das Reaktionsgemisch verrührt, und die organische Phase wurde abgetrennt. Die wäßrige Phase wurde noch zweimal mit je 50 ml Methylenchlorid extrahiert, und die vereinigten organischen Phasen wurden mit 1N wäßriger Natronlauge und Wasser gewaschen und über Natriumsulfat getrocknet. Man erhielt 51,6 g (74,3 % der Theorie) 5-(4-Aminophenoxy)-3-iso-propyl-1,2,4-thiadiazol vom Schmelzpunkt 82°C - 85°C.

Beispiel 15

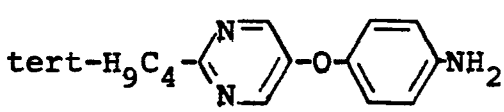

(II-2)

80 g (0,293 Mol) 2-tert-Butyl-5-(4-nitrophenoxy)-pyrimidin wurden in 800 ml Methanol mit 11 g Raney-Nickel als Katalysator unter einem Wasserstoff-Druck von 100 atm innerhalb von 7 Stunden bei 60°C hydriert. Das Raney-Nickel wurde abgesaugt, mit Methanol nachgewaschen, und das Filtrat wurde eingedampft.

Man erhielt 68 g (95,5 % der Theorie) 5-(4-Aminophenoxy)-2-tert-butyl-pyrimidin vom Schmelzpunkt 66°C - 69°C.

Vorprodukte der Formel (V)

Beispiel 16

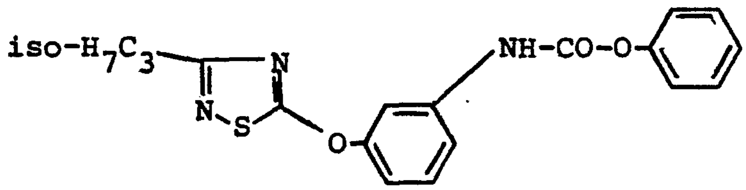

(V-1)

35,3 g (0,15 Mol) 5-(3-Aminophenoxy)-3-iso-propyl-1,2,4-thiadiazol wurden in 170 ml Methylenchlorid und 12 ml Pyridin vorgelegt und tropfenweise mit 21 ml (0,186 Mol) Chlorameisensäurephenylester versetzt. Anschließend wurde 10 Stunden unter Rückfluß erhitzt, auf 25°C abgekühlt und in 150 ml 1 n wäßrige Salzsäure eingerührt. Die organische Phase wurde abgetrennt, mit Wasser gewaschen, über Natriumsulfat getrocknet und eingeengt. Der ölige Rückstand wurde mit n-Hexan zur Kristallisation gebracht. Das kristalline Produkt wurde abgesaugt und bei 50°C getrocknet.

Man erhielt 49,4 g (92,6 % der Theorie) N-/3-(3-iso-Propyl-1,2,4-thiadiazol-5-yloxy)-phenyl/-carbamidsäurephenylester vom Schmelzpunkt 96°C - 98°C.

Vorprodukte der Formel (XII)

Beispiel 17

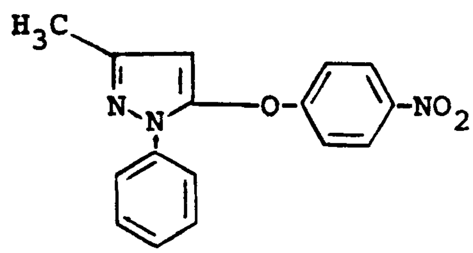

(XII-1)

156,6 g (0,9 Mol) 5-Hydroxy-3-methyl-1-phenylpyrazol wurden mit 40 g (1 Mol) festem Natriumhydroxid in 750 ml Dimethylsulfoxid 30 Minuten bei 120°C gerührt. Anschließend wurden 137 g (0,87 Mol) 4-Chlor-nitrobenzol dazugegeben. Es wurde 7 Stunden bei 120°C gerührt, mit 8 g (0,2 Mol) Natriumhydroxid und 31,4 g (0,2 Mol) 4-Chlor-nitrobenzol versetzt und weitere 15 Stunden bei 120°C gerührt. Das Reaktionsgemisch wurde auf 25°C abgekühlt, in 1,5 l Wasser eingerührt, mit wäßriger Salzsäure auf pH1 gestellt und mit Methylenchlorid extrahiert. Die organische Phase wurde eingeengt, und der Rückstand in 2 l 1n wäßrige Natronlauge eingerührt. Der unlösliche Anteil wurde abgesaugt, mit wenig 1n wäßriger Natronlauge und Wasser gewaschen und bei 80°C getrocknet.

Man erhielt 105,9 g (40 % der Theorie) 3-Methyl-5-(4-nitro-phenoxy)-1-phenylpyrazol vom Schmelzpunkt 90°C - 100 °C.

Beispiel 18

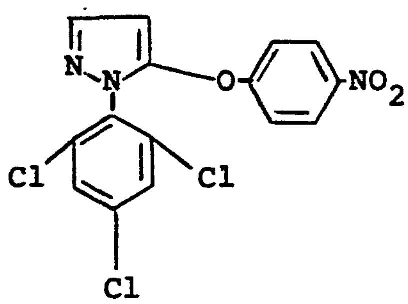

(XII-2)

Fp: 161°C - 163°C

Verwendungsbeispiele

In dem nachfolgend beschriebenen biologischen Test wurde die folgende Verbindung als Vergleichssubstanz eingesetzt.

(A) 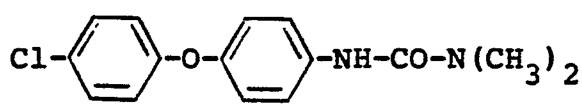

1,1-Dimethyl-3-/4-(4-chlorphenoxy)-phenyl7-harnstoff

(bekannt aus DE-AS 1 142 251)

Beispiel A

Post-emergence-Test/Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der

angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigte der erfindungsgemäße Wirkstoff (I-1) bei der Bekämpfung von Unkräutern in Mais eine bessere selektive herbizide Wirksamkeit als die Vergleichssubstanz (A).

Patentansprüche

1) Substituierte Phenylharnstoffe der Formel

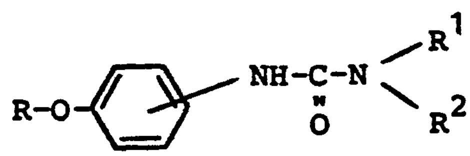

in welcher

R für gegebenenfalls substituierte Reste aus der Reihe Pyrimidinyl, Pyrazolyl, 1,2,4-Triazolyl, Thiazolo-/1,2,4/-triazolyl, 1,2,4-Oxadiazolyl, 1,2,4-Thiadiazolyl, Imidazolyl, 1,3-Oxazolyl oder 1,3-Thiazolyl steht,

$R^1$ für Wasserstoff oder Alkyl steht und

$R^2$ für Alkyl steht.

2) Substituierte Phenylharnstoffe der Formel (I), in denen

R für 5-Pyrimidinyl oder 6-Pyrimidinyl steht, wobei jeder dieser Reste einfach oder mehrfach, gleichartig oder verschieden substituiert sein kann durch gegebenenfalls substituiertes Alkyl mit 1 bis 6 Kohlenstoffatomen, Alkoxy mit 1 bis 6 Kohlenstoffatomen, Alkylthio mit 1 bis 6 Kohlenstoffatomen, Cycloalkyl mit 3 bis 8 Kohlenstoffatomen, Amino, Alkylamino mit 1 bis 4 Kohlenstoffatomen, Dialkylamino mit 1 bis 4 Kohlenstoffatomen in jeder Alkylgruppe und/oder gegebenenfalls substituiertes Phenyl, oder

R für Pyrazolyl steht, das einfach oder zweifach, gleichartig oder verschieden substituiert sein kann durch Alkyl mit 1 bis 4 Kohlenstoffatomen, Cycloalkyl mit 3 bis 8 Kohlenstoffatomen und/oder Phenyl, welches seinerseits einfach bis dreifach, gleichartig oder verschieden substituiert sein kann durch Alkyl mit 1 oder 2 Kohlenstoffatomen, Alkoxy mit 1 oder 2 Kohlenstoffatomen, Fluor, Chlor, Brom, Trihalogenmethyl, Trihalogenmethoxy, Dihalogenmethyl und/oder Trihalogenmethylsulfonyl,

oder

R für 1,2,4-Triazolyl oder Thiazolo-[1,2,4]-triazolyl steht, wobei jeder dieser Reste einfach oder zweifach, gleichartig oder verschieden substituiert sein kann durch Alkyl mit 1 bis 4 Kohlenstoffatomen und/oder gegebenenfalls substituiertes Phenyl,

oder

R für 1,2,4-Oxadiazolyl, 1,2,4-Thiadiazolyl, Imidazolyl, 1,3-Oxazolyl oder 1,3-Thiazolyl steht, wobei jeder dieser Reste einfach bis dreifach, gleichartig oder verschieden substituiert sein kann durch Alkyl mit 1 bis 4 Kohlenstoffatomen und/oder Halogen,

$R^1$ für Wasserstoff oder Alkyl mit 1 bis 6 Kohlen-

stoffatomen steht und

$R^2$ für Alkyl mit 1 bis 6 Kohlenstoffatomen steht.

3) Verfahren zur Herstellung von substituierten Phenylharnstoffen der Formel

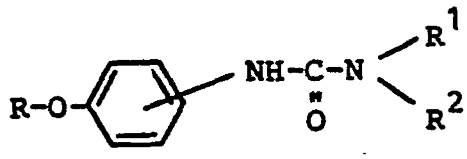

(I)

in welcher

R für gegebenenfalls substituierte Reste aus der Reihe Pyrimidinyl, Pyrazolyl, 1,2,4-Triazolyl, Thiazolo-[1,2,4]-triazolyl, 1,2,4-Oxadiazolyl, 1,2,4-Thiadiazolyl, Imidazolyl, 1,3-Oxazolyl oder 1,3-Thiazolyl steht,

$R^1$ für Wasserstoff oder Alkyl steht und

$R^2$ für Alkyl steht,

dadurch gekennzeichnet, daß man

a) substituierte Phenylamine der Formel

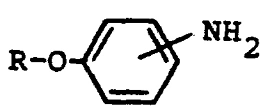

(II)

in welcher

R die oben angegebene Bedeutung hat,

entweder

α) mit Carbamoylhalogeniden der Formel

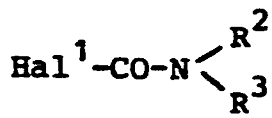 (III)

in welcher

$R^2$ die oben angegebene Bedeutung hat,

$R^3$ für Alkyl steht und

$Hal^1$ für Halogen steht,

gegebenenfalls in Gegenwart eines Säureakzeptors und gegebenenfalls in Gegenwart eines Verdünnungsmittels umsetzt,

oder

ß) mit Isocyanaten der Formel

$R^2$-NCO (IV)

in welcher

$R^2$ die oben angegebene Bedeutung hat,

gegebenenfalls in Gegenwart eines Katalysators und gegebenenfalls in Gegenwart eines Verdünnungsmittels umsetzt,

b) Carbamidsäure-Derivate der Formel

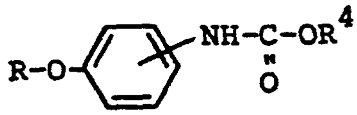 (V)

in welcher

R die oben angegebene Bedeutung hat und

$R^4$ für gegebenenfalls substituiertes Alkyl oder gegebenenfalls substituiertes Phenyl steht,

mit Aminen der Formel

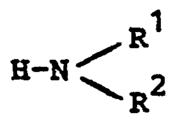 (VI)

in welcher

$R^1$ und $R^2$ die oben angegebene Bedeutung haben,

gegebenenfalls in Gegenwart eines Verdünnungsmittels umsetzt,

oder

c) substituierte Phenylamine der Formel

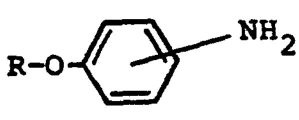 (II)

in welcher

R die oben angegebene Bedeutung hat,

zunächst mit Phosgen in Gegenwart eines Verdünnungsmittels umsetzt und die dabei enstehenden Isocyanate der Formel

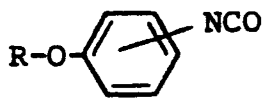

(VII)

in welcher

R die oben angegebene Bedeutung hat,

mit Aminen der Formel

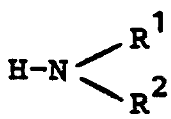

(VI)

in welcher

$R^1$ und $R^2$ die oben angegebene Bedeutung haben,

gegebenenfalls in Gegenwart eines Katalysators und gegebenenfalls in Gegenwart eines Verdünnungsmittels umsetzt.

4) Herbizide Mittel, gekennzeichnet durch einen Gehalt an mindestens einem substituierten Phenylharnstoff der Formel (I).

5) Verwendung von substituierten Phenylharnstoffen

der Formel (I) zur Bekämpfung von Unkräutern.

6) Verfahren zur Bekämpfung von Unkräutern, dadurch gekennzeichnet, daß man substituierte Phenylharnstoffe der Formel (I) auf Unkräuter und/oder deren Lebensraum ausbringt.

7) Verfahren zur Herstellung von herbiziden Mitteln, dadurch gekennzeichnet, daß man substituierte Phenylharnstoffe der Formel (I) mit Streckmitteln und/oder oberflächenaktiven Stoffen vermischt.

8) Substituierter Phenylharnstoff der Formel

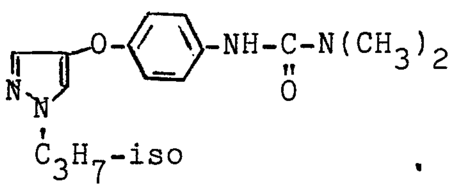

,

9) Substituierter Phenylharnstoff der Formel

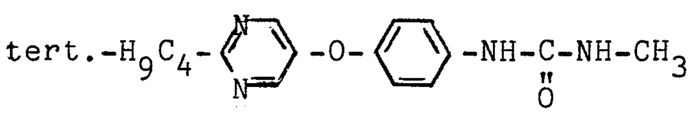

,

10) Substituierter Phenylharnytoff der Formel

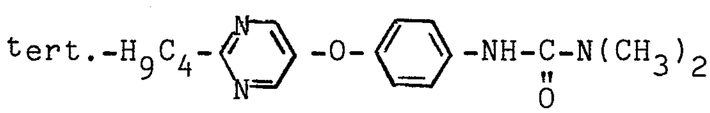

,

Europäisches Patentamt

# EUROPÄISCHER RECHERCHENBERICHT

0177710

Nummer der Anmeldung

EP 85 11 0066

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | DE-A-3 220 822 (KONISHIROKU)<br>--- | | C 07 D 231/22<br>C 07 D 231/18<br>C 07 D 231/20<br>C 07 D 239/34<br>C 07 D 285/08<br>C 07 D 513/04<br>C 07 D 249/12<br>C 07 D 271/06<br>C 07 D 233/70<br>C 07 D 263/38<br>C 07 D 277/34 |
| A | EP-A-0 001 187 (ICI)<br>--- | | |
| A | GB-A-2 093 840 (ROUSSEL-UCLAF)<br>--- | | |
| P,A | EP-A-0 123 861 (BAYER)<br>----- | | |
| | | | RECHERCHIERTE SACHGEBIETE (Int. Cl 4) |
| | | | C 07 D 231/00<br>C 07 D 239/00<br>C 07 D 285/00<br>C 07 D 513/00<br>C 07 D 249/00<br>C 07 D 271/00<br>C 07 D 233/00<br>C 07 D 263/00<br>C 07 D 277/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 03-01-1986 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument